# EUROPEAN PATENT SPECIFICATION

(11) **EP 3 423 030 B1**
(45) Date of publication and mention of the grant of the patent: **24.04.2024**
(21) Application number: 17760588.8
(22) Date of filing: 28.02.2017
(51) Int. Cl.: A61K 8/38, A61K 31/192, A61K 31/203, A61K 31/327, A61K 8/11, A61Q 17/04, A61Q 19/00

(54) **ENCAPSULATION OF PEROXIDES FOR SKIN APPLICATIONS**
VERKAPSELUNG VON PEROXIDEN FÜR HAUTANWENDUNGEN
ENCAPSULATION DE PEROXYDES POUR APPLICATIONS CUTANÉES

(30) Priority: 04.03.2016 US 201662303416 P
(43) Date of publication of application: 09.01.2019
(73) Proprietor: Arkema, Inc., King of Prussia, PA 19406 (US)
(72) Inventor: DLUZNESKI, Peter R., Harleysville PA 19438 (US); SALVADOR, Tomas, Sugar Land TX 77479 (US); KOZEL, Thomas H., Pottstown PA 19465 (US)
(74) Representative: Bandpay & Greuter
(86) International application number: PCT/US2017/019894
(87) International publication number: WO 2017/151584

(56) References cited:
- WO-A1-03/039510
- WO-A1-2012/170866
- WO-A2-2008/093346
- WO-A2-2011/080741
- US-A1- 2003 178 600
- US-A1- 2004 101 566
- US-A1- 2009 124 693
- US-A1- 2012 015 014
- US-A1- 2012 064 135
- US-B1- 8 697 130

## Description

### FIELD OF THE INVENTION

The present invention relates to methods for encapsulating solid peroxides, such as benzoyl peroxide, and products made by those methods.

### BACKGROUND OF THE INVENTION

Benzoyl peroxide (BPO) is commonly used for the treatment of skin disorders, such as acne, seborrhea and skin infections. Acne vulgaris is a well-known skin condition, most common during adolescence, and is characterized by noninflammatory follicular papules or comedones and by inflammatory papules, pustules, and nodules in its more severe forms. The areas more affected by acne are the face, the upper part of the chest, and the back.

Benzoyl peroxide, (C₆H₅CO)₂O₂, is a solid that is stable at room temperature. It is a powerful oxidizing agent that is non-toxic and has been employed as an effective anti-bacterial and keratolytic agent in the treatment of acne. However, benzoyl peroxide routinely causes dryness, local irritation and redness, and can cause excessive skin irritation. Its poor water solubility, coupled with its chemical instability in other solvents, presents challenges with respect to formulating topical products with optimal bioavailability, stability, and tolerability.

There have been previous attempts to provide suitable vehicles for benzoyl peroxide; however, many of them are not particularly suitable for use in skin applications. U.S. Patent No. 5,409,764 describes the preparation of microencapsulated benzoyl peroxide for use in curable adhesive formulations. U.S. Patent No. 3,839,220 describes microcapsules containing benzoyl peroxide with gelatin shells. U.S. Publication No. 2012/0258177 describes benzoyl peroxide microcapsules produces by solvent evaporation techniques with acrylic or cellulose shells. Encapsulation of benzoyl peroxide using sol-gel has also been demonstrated using inorganic shell materials such as silica (U.S. Patent No. 8,449,918) and metal/silicon alkoxides and metal esters (U.S. Publication No. 2013/0095185). U.S. Publication No. US2012/0064135 describes a method that encapsulates or isolates benzoyl peroxide within a fatty substance such as cocoa butter. U.S. Patent Nos. 6,699,403, 6,764,612 and 6,843,935 also relate to encapsulated peroxides.

There remains a need for formulations that can deliver peroxides and other beneficial ingredients to the skin, while inhibiting inflammation and other skin irritations.

### SUMMARY OF THE INVENTION

Embodiments of the present invention relate to processes for encapsulating benzoyl peroxide, and compositions made by such processes. Embodiments of the invention also relate to methods of using the encapsulated benzoyl peroxide compositions for topical skin applications.

Benzoyl peroxide is commonly used for the treatment of acne and other skin disorders; however, a major drawback of benzoyl peroxide is the common incurrence of inflammation, as well as dryness, irritation and redness. The inventors have discovered a method of encapsulating a peroxide, which decreases the rate at which the peroxide is released to the skin, so that inflammation is inhibited. The inventors have also discovered that the peroxide can be encapsulated in materials that have beneficial effects on the skin, and that further inhibit inflammation (for example, retinoate salts and other fatty acid salts, such as salts of omega-3 and omega-6 fatty acids).

Embodiments of the present invention relate to a method of encapsulating a solid peroxide comprising:
dispersing solid peroxide particles in an aqueous dispersion of one or more monovalent fatty acid salts to form a peroxide/fatty acid dispersion, wherein the solid peroxide particles have a mean particle size (D50) of 10 µm or less as measured by laser light scattering; and
subsequently encapsulating the solid peroxide particles by reacting the one or more monovalent fatty acid salts with one or more multivalent salts of calcium, magnesium, aluminum, silver, and/or zinc, thereby forming encapsulated peroxide particles, wherein the peroxide is benzoyl peroxide.

Embodiments of the present invention also relate to a peroxide microparticle comprising one or more solid peroxide particles encapsulated within one or more fatty acid salts, wherein the peroxide is benzoyl peroxide, wherein the one or more solid peroxide particles have a mean particle size (D50) of 10 µm or less, and wherein the one or more fatty acid salts are selected from the group consisting of salts of omega-3 fatty acids, salts of omega-6 fatty acids, salts of gamma-linolenic acid, salts of retinoic acid, salts of ricinoleic acid, salts of caprylic acid, salts of capric acid, salts of lauric acid, salts of myristic acid, salts of palmitic acid, salts of stearic acid, salts of arachidic acid, salts of behenic acid, salts of erucic acid, salts of lignostearic acid, salts of cerotic acid, salts of oleic acid, salts of elaidic acid, salts of linoleic acid, salts of undecylinic acid, salts of alpha-linolenic acid, salts of arachidonic acid, salts of ascorbic acid, salts of eleostearic acid, and combinations thereof, said salts being salts of calcium, magnesium, aluminum, silver, and/or zinc.

Embodiments of the present invention also relate to topical compositions comprising the peroxide microparticles (*e.g*., gels, creams and lotions), and to methods of using the topical compositions. For example, a method of treating a skin disorder comprises applying the topical composition to an affected area of skin.

### DETAILED DESCRIPTION

The inventors have discovered methods of encapsulating a solid peroxide which decrease the rate at which the peroxide is released to the skin, so that inflammation is inhibited. In particular, the inventors have discovered that a reduction in inflammation can be achieved by reducing the particle size of the peroxide, preferably to a diameter mean particle size (D50) that is less than 10 µm, and by encapsulating the fine particles in fatty acid salts that have beneficial impacts on the skin (*e.g*., retinoate salts and salts of other fatty acids, such as salts of omega-3 and omega-6 fatty acids). In accordance with particular embodiments, the release rate of the peroxide is delayed over a longer period of time than a non-encapsulated version, and the encapsulating materials comprise salts of fatty acids that are not inert but may have beneficial effects on the skin to which they are applied.

According to particular embodiments, the method of the present invention involves the formation of micelles of sodium or potassium salts of fatty acids (fatty acid soaps) around solid peroxide particles benzoyl peroxide), wherein the peroxide particles are less than 10 microns in diameter mean particle size (D50). This may be accomplished by dissolving the fatty acid sodium/potassium soap in a dispersion of the peroxide or by adding solid peroxide or an aqueous dispersion of solid peroxide to a pre-made aqueous dispersion of fatty acid sodium/potassium soap. A micelle is a spherical aggregate of lipid molecules in an aqueous medium arranged so that the hydrophilic heads of the lipid molecules form the exterior surface of the sphere and the hydrophobic tails of the lipid molecules pack the inside of the sphere. The micelles are then reacted with multivalent salts of calcium, magnesium, aluminum, silver, or zinc to form insoluble salts of the fatty acids which surround the peroxide particles. In the case of the present invention, the hydrophobic peroxide particles are believed to be located inside the spheres, packed in with the lipid tails of the lipid molecules (the fatty acid salts). These fatty acid salts (calcium, magnesium, aluminum silver or zinc salts of fatty acids) are relatively insoluble in solvents (e.g., water), resulting in encapsulation of the peroxide. According to certain embodiments, this encapsulation results in retarding the rate of release of the peroxide into the cutaneous area in which it is applied, limiting the immediate impact of the peroxide on the skin tissue and providing a time-released effect.

Beneficial fatty acids can be used in the encapsulant (*i.e*., the material surrounding the benzoyl peroxide particles), which provide additional effects on the skin. For example, gamma-linolenic acid has been found to be an anti-inflammatory agent which can counteract the potential inflammatory effect of the peroxide. Additional examples of such materials include retinoic acid, which is known to have beneficial effects on skin; ricinoleic acid, which is known for its moisturizing effect on the skin; and omega-3 and omega-6 fatty acids. These fatty acids may be pre-dispersed in water as soluble sodium or potassium salts prior to addition of the solid peroxide or an aqueous dispersion of the peroxide, and precipitated with an appropriate soluble salt of calcium, magnesium, aluminum, silver, or zinc to form insoluble encapsulating salts of the fatty acids. Examples of soluble salts that may be used include, but are not limited to halide, nitrate, sulfate and carboxylate salts such as calcium chloride, calcium nitrate, calcium acetate, magnesium chloride, magnesium sulfate, magnesium acetate, magnesium citrate, magnesium nitrate, aluminum nitrate, aluminum sulfate, silver nitrate, zinc chloride, zinc nitrate, zinc sulfate, zinc acetate, and zinc nitrate and combinations thereof. The metal cation that is used to react with the fatty acids to form the encapsulating film may also provide beneficial effects to the skin. For example, zinc has been shown to prevent and alleviate inflammation and scarring associated with acne, and is widely used in lotions to soothe and heal diaper rash. Magnesium is believed to be effective in reducing the inflammation caused by acne, and calcium is used in the epidermis to make sebum, which moisturizes the skin. Additional ingredients that can have a beneficial impact on the skin may be co-encapsulated with the peroxide, such as squalene, tocopherols, and finely ground elemental sulfur.

According to particular embodiments, the process occurs as a "single encapsulation" in which the peroxide and other ingredients are contained within a single film coating. Alternatively, the product can be the result of successive encapsulations in which a multi-layer particle may be formed to deliver varying time-release results. Non-irritating non-ionic surfactants may also be included in the process to maximize aqueous dispersibility and provide further phase stabilization of aqueous pastes containing the encapsulated peroxide particles.

One of the advantages of this encapsulation system is that the fatty acids are similar to the oil composition found in the pilosebaceous unit on the skin. Unlike other encapsulation materials, embodiments of the compositions described herein are not foreign to the biochemistry of the skin. The biochemical similarity of the encapsulation may result in better targeted deposition, facile penetration, and a longer duration in the pilosebaceous unit. Selection of the proper fatty acids, cationic precipitants, and additional ingredients can minimize inflammation, increase blood flow, stimulate new cell growth, and augment moisturizing of the skin.

One aspect of the invention relates to a method of encapsulating a solid peroxide, wherein the method comprises, consists essentially of, or consists of:
dispersing solid peroxide particles in a solvent to form a peroxide dispersion, wherein the peroxide particles have diameter mean particle size (D50) of 10 µm or less;
dissolving one or more monovalent fatty acid salts in the solvent to form a peroxide/fatty acid salt dispersion; and
subsequently encapsulating the solid peroxide particles by reacting the one or more monovalent fatty acid salts with one or more multivalent salts of calcium, magnesium, aluminum, silver, and/or zinc, thereby forming encapsulated peroxide particles.

The solid peroxide particles comprise, consist essentially of, or consist of benzoyl peroxide.

The method may further comprise reducing the particle size of the solid peroxide particles prior to dispersing them in the solvent. According to particular embodiments, the particle sizes of the solid peroxide particles are reduced according to methods described in U.S. Publication No. 2015/0165043 and U.S. Publication No. 2013/0344152. As described in U.S. Publication No. 2015/0165043 and U.S. Publication No. 2013/0344152, the viscosity of aqueous dispersions of solid organic peroxides may be lowered through the use of surfactants which are polyglyceryl esters of C6-C12 fatty acids. The reduction in viscosity facilitates milling of the peroxides to reduce particle size, and provides dispersions of peroxides with small particle sizes.

According to particular embodiments, the mean particle size (D50) of the peroxide particles is reduced to 10 µm or less as measured by laser light scattering (a standard particle sizing technique), or to 8 µm or less, or to 6 µm or less, or to 4 µm or less, or to 2 µm or less, or to 1 µm or less. For example, the mean particle size (D50) of the peroxide particles may be between 0.1 µm and 10 µm, or between 0.1 µm and 8 µm, or between 0.1 µm and 6 µm, or between 0.1 µm and 4 µm, or between 0.5 µm and 10 µm, or between 0.5 µm and 8 µm, or between 0.5 µm and 6 µm, or between 0.5 µm and 4 µm, or between 1 µm and 10 µm, or between 1 µm and 8 µm, or between 1 µm and 6 µm, or between 1 µm and 4 µm. The reduced particle size increases efficacy by enabling the peroxide particles to effectively penetrate pores of the skin. Particle sizes within these ranges also allow the benzoyl peroxide to be more thoroughly dispersed in a carrier, making it easier to distribute the benzoyl peroxide particles evenly across the area of application and to be more easily introduced into the affected pores.

The solid peroxide particles may be dispersed in any suitable solvent to form the peroxide dispersion. According to particular embodiments, the solvent is water. Alternatively, the solvent may include water and optionally one or more water-miscible organic solvents (*e.g*., one or more solvents selected from the group consisting of alcohols, glycols, glycol ethers, esters, ketones, and a combination thereof).

According to particular embodiments, the monovalent fatty acid salt(s) that are dissolved in the peroxide dispersion to form the peroxide/fatty acid salt dispersion comprise one or more fatty acid salts that are beneficial to human skin (*e.g*., they provide anti-inflammatory effects).

Non-limiting examples of such fatty acid salts include salts of omega-3 fatty acids, salts of omega-6 fatty acids, salts of gamma-linolenic acid, salts of retinoic acid, salts of ricinoleic acid, salts of caprylic acid, salts of capric acid, salts of lauric acid, salts of myristic acid, salts of palmitic acid, salts of stearic acid, salts of arachidic acid, salts of behenic acid, salts of erucic acid, salts of lignostearic acid, salts of cerotic acid, salts of oleic acid, salts of elaidic acid, salts of linoleic acid, salts of undecylinic acid, salts of alpha-linolenic acid, salts of arachidonic acid, salts of ascorbic acid, salts of eleostearic acid, and combinations thereof. The fatty acid of the fatty acid salt may be a saturated fatty acid, an unsaturated fatty acid, a polyunsaturated fatty acid or a combination thereof.

Preferred monovalent fatty acid salts include salts of the following fatty acids: caprylic acid, capric acid, lauric acid, myristic acid, palmitic acid, stearic acid, arachidic acid, aehenic acid, erucic acid, lignostearic acid, cerotic acid, oleic acid, elaidic acid, linoleic acid, undecylinic acid, alpha-linolenic acid, gamma-linolenic acid, arachidonic acid, ascorbic acid, retinoic acid, ricinoleic acid, eleostearic acid, other omega-3 fatty acids (such as eicosapentaenoic acid and docosahexaenoic acid), and other omega-6 fatty acids (such as calendic acid, eicosadienoic acid, dihomo-gamma-linoleic acid, docosadienoic acid, adrenic acid, docosapentaenoic acid, tetracosatetraenoic acid and tetracosapentaenoic acid) and combinations thereof.

More preferred monovalent fatty acid salts include salts of the following fatty acids: myristic acid, palmitic acid, oleic acid, ricinoleic acid, lauric acid, stearic acid, linoleic acid, linolenic acids, retinoic acid, omega-3 fatty acids, and omega-6 fatty acids and combinations thereof."

For example, the salts may be sodium and/or potassium salts of the aforementioned fatty acids.

The amount of monovalent fatty acid salt used will vary somewhat depending upon the particle size of the solid peroxide, the amount of solid peroxide added to the dispersion and the critical micelle concentration of the monovalent fatty acid salt, among other possible factors. In certain embodiments of the invention the weight ratio of monovalent fatty acid salt to solid peroxide may be from about 1:1 to about 1:50, or from about 1:5 to about 1:35 or from about 1:8 to about 1:25.

According to particular embodiments, the method further comprises a step of homogenizing the peroxide/fatty acid salt dispersion, wherein the homogenization causes the monovalent fatty acid salts to form micelles around the solid peroxide particles. The homogenization step also inhibits the peroxide particles from agglomerating together. According to particular embodiments, monovalent fatty acid salts may form a micelle around a single peroxide particle. The solid peroxide particles are then encapsulated by reacting the micelles with one or more multivalent salts of calcium, magnesium, aluminum, silver, and/or zinc (also referred to herein as "cationic precipitants"). Preferred multivalent salts include silver, aluminum, calcium, magnesium and zinc salts and combinations thereof. More preferred multivalent salts include aluminum, calcium, magnesium and zinc salts and combinations thereof. Most preferred multivalent salts include calcium, magnesium and zinc salts and combinations thereof. The fatty acid salts surrounding the peroxide particles are precipitated out of solution because the multivalent salts render the fatty acid salts insoluble. Thus, the peroxide particles, which have a mean particle size (D50) of 10 µm or less, become encapsulated by the multivalent fatty acid salts. Stated another way, the fatty acid salts surround or enclose the peroxide particles (in some cases, the fatty acid salts surround or enclose a single peroxide particle). The encapsulated peroxide particles may be referred to as "microparticles" that have a core-shell structure, wherein the peroxide particles form the core, and they are surrounded by a "shell" of fatty acid salts. As discussed herein, this encapsulation limits the immediate impact of the peroxide on the skin tissue and provides a time-released effect.

The amount of multivalent salt used is, in certain embodiments, an amount effective to convert all or substantially all (e.g., at least 80% or at least 90%) of the monovalent fatty acid salt present in the aqueous dispersion to multivalent fatty acid salt. Thus, in certain embodiments of the invention, the amount of multivalent salt combined with the aqueous dispersion is an amount which is at least 80%, at least 90% or at least 100% of the calculated stoichiometric amount needed to replace the monovalent cations of the monovalent fatty acid salts (e.g., Na⁺, K⁺) with multivalent cations of the multivalent salt (e.g., Ca⁺², Z⁺², Mg⁺²). A stoichiometric excess of multivalent salt relative to monovalent fatty acid salt may be employed. The encapsulated peroxide particles obtained may, in certain embodiments, contain little or no residual monovalent fatty acid salt (e.g., less than 20% by weight or less than 10% by weight or even 0% by weight monovalent fatty acid salt, based on the total weight of fatty acid salt).

Although the multivalent salt(s) could be combined with the aqueous dispersion in solid form, in certain embodiments of the invention the multivalent salt is in the form of a solution (in particular, in the form of an aqueous solution) at the time of combination. The concentration of multivalent salt in solution is not believed to be critical, but may for example be from about 0.1 to about 5 M. In certain embodiments, a solution of multivalent salt is added to the aqueous dispersion, either all at once or incrementally or in portions. The aqueous dispersion may be stirred or otherwise mixed or agitated when the multivalent salt and aqueous dispersion are combined. Combining the multivalent salt and aqueous dispersion may be conducted at any suitable temperature; in certain embodiments, temperatures around room temperature (e.g., 15°C to 35°C) are employed.

According to particular embodiments, the method further comprises filtering the encapsulated peroxide particles out of solution, which may remove excess cationic precipitants or surfactants. The encapsulated peroxide particles (or "peroxide microparticles") are solid, and may be re-suspended in another aqueous vehicle, such as water, or another type of vehicle, such as a cream, gel or lotion.

Another aspect of the invention relates to a peroxide microparticle comprising one or more solid peroxide particles encapsulated within one or more multivalent fatty acid salts, wherein the one or more solid peroxide particles have mean particle size (D50) of 10 µm or less, and wherein the one or more fatty acid salts are selected from the group consisting of multivalent salts of salts of omega-3 fatty acids, salts of omega-6 fatty acids, salts of gamma-linolenic acid, salts of retinoic acid, salts of ricinoleic acid, salts of caprylic acid, salts of capric acid, salts of lauric acid, salts of myristic acid, salts of palmitic acid, salts of stearic acid, salts of arachidic acid, salts of behenic acid, salts of erucic acid, salts of lignostearic acid, salts of cerotic acid, salts of oleic acid, salts of elaidic acid, salts of linoleic acid, salts of undecylinic acid, salts of alpha-linolenic acid, salts of arachidonic acid, salts of ascorbic acid, salts of eleostearic acid, and combinations thereof.

Another aspect of the invention relates to topical compositions comprising a plurality of peroxide particles of the present invention. For example, the peroxide particles may be formulated in creams, gels, lotions or other carriers for skin treatment. The carrier can be any carrier typically used in the cosmetic or topical pharmaceutical fields. One or more additives that are common in topical formulations may also be present, including, without limitation: thickeners, preservatives, antioxidants, fragrances, emulsifiers, moisturizing agents, emollients, sequestering agents, surfactants, fillers, sunscreen agents, colorants, and combinations thereof.

Another aspect of the present invention relates to a method of using the encapsulated peroxide particles comprising applying the encapsulated peroxide particles to human skin; for example, by rubbing a cream, gel or lotion comprising the peroxide particles into the skin. Another aspect of the present invention relates to a method of treating a skin disorder (*e.g*., acne, seborrhea or skin infections) comprising applying the encapsulated peroxide particles to skin; for example, by rubbing a cream, gel or lotion comprising the peroxide particles into the skin. The peroxide particles are preferably applied directly to one or more affected areas of the skin, *i.e.,* areas that are affected by skin disorders, such as acne, seborrhea and skin infections.

The embodiments described herein are intended to be exemplary of the invention and not limitations thereof. The embodiments of the invention are described above using the term "comprising" and variations thereof. However, it is the intent of the inventors that the term "comprising" may be substituted in any of the embodiments described herein with "consisting of" and "consisting essentially of" without departing the scope of the invention.

The following examples further illustrate the best mode contemplated by the inventors for the practice of their invention and are to be construed as illustrative and not in limitation thereof.

### EXAMPLES

### Example 1

An emulsion was formed by stirring 0.2g of potassium stearate into 50 mL of water. 3g of Luperox^{®} A75 BPO (benzoyl peroxide) was added and stirred for an additional hour. 0.25g of a 4.8M CaCl₂ solution was added, rapidly precipitating the mixture. After settling for several hours, a clear supernatant is observed.

### Example 2

An emulsion was formed by stirring 0.2g of potassium stearate into 50 mL of water. 3g of Luperox^{®} A75 BPO was added and stirred for an additional hour. 0.4g of a 1.8M Zn Acetate solution was added to the mixture, rapidly precipitating the mixture. After settling for several hours, a clear supernatant is observed.

### Example 3

An emulsion was formed by stirring 0.2g of sodium oleate into 50 mL of water. 3g of Luperox^{®} A75 BPO was added and stirred for an additional hour. 1g of a 1.8M Zn Acetate solution was added to the mixture, rapidly precipitating the mixture. After settling for several hours, a clear supernatant is observed.

### Example 4

An emulsion was formed by stirring 0.2g of potassium ricinoleate into 50 mL of water. 3g of Luperox^{®} A75 BPO was added and stirred for an additional hour. 0.5g of a 1.8M Zn Acetate solution was added to the mixture, rapidly precipitating the mixture. After settling for several hours, a clear supernatant is observed.

## Claims

1. A method of encapsulating a solid benzoyl peroxide comprising:
- dispersing solid benzoyl peroxide particles in an aqueous dispersion of one or more monovalent fatty acid salts to form a benzoyl peroxide/monovalent fatty acid salt dispersion, wherein the benzoyl peroxide particles have diameter mean particle size (D50) of 10 µm or less; and
- subsequently encapsulating the solid benzoyl peroxide particles by reacting the one or more monovalent fatty acid salts with one or more multivalent salts of calcium, magnesium, aluminum, silver, and/or zinc, thereby forming encapsulated solid peroxide particles of benzoyl peroxide.

2. The method of claim 1, further comprising homogenizing the peroxide/monovalent fatty acid salt dispersion, wherein the homogenization causes the monovalent fatty acid salts to form micelles around the solid peroxide particles.

3. The method of claim 1 or 2, wherein the monovalent fatty acid salts are selected from the group consisting of sodium and potassium salts of omega-3 fatty acids, sodium and potassium salts of omega-6 fatty acids, sodium and potassium salts of gamma-linolenic acid, sodium and potassium salts of retinoic acid, sodium and potassium salts of ricinoleic acid, sodium and potassium salts of caprylic acid, sodium and potassium salts of capric acid, sodium and potassium salts of lauric acid, sodium and potassium salts of myristic acid, sodium and potassium salts of palmitic acid, sodium and potassium salts of stearic acid, sodium and potassium salts of arachidic acid, sodium and potassium salts of behenic acid, sodium and potassium salts of erucic acid, sodium and potassium salts of lignostearic acid, sodium and potassium salts of cerotic acid, sodium and potassium salts of oleic acid, sodium and potassium salts of elaidic acid, sodium and potassium salts of linoleic acid, sodium and potassium salts of undecylinic acid, sodium and potassium salts of alpha-linolenic acid, sodium and potassium salts of arachidonic acid, sodium and potassium salts of ascorbic acid, sodium and potassium salts of eleostearic acid, and combinations thereof.

4. The method of any one of claims 1 to 3, wherein the mean particle size (D50) of the peroxide particles is between 0.1 µm and 10 µm, and preferably between 0.1 µm and 4 µm.

5. Encapsulated peroxide particles made according to the method of any one of claims 1 to 4.

6. A benzoyl peroxide microparticle comprising:
- one or more solid benzoyl peroxide particles encapsulated within one or more multivalent fatty acid salts,
- wherein the one or more solid benzoyl peroxide particles have diameter mean particle size (D50) of 10 µm or less, and
- wherein the one or more multivalent fatty acid salts are selected from the group consisting of salts of omega-3 fatty acids, salts of omega-6 fatty acids, salts of gamma-linolenic acid, salts of retinoic acid, salts of ricinoleic acid, salts of caprylic acid, salts of capric acid, salts of lauric acid, salts of myristic acid, salts of palmitic acid, salts of stearic acid, salts of arachidic acid, salts of behenic acid, salts of erucic acid, salts of lignostearic acid, salts of cerotic acid, salts of oleic acid, salts of elaidic acid, salts of linoleic acid, salts of undecylinic acid, salts of alpha-linolenic acid, salts of arachidonic acid, salts of ascorbic acid, salts of eleostearic acid, and combinations thereof, said salts being salts of calcium, magnesium, aluminum, silver, and/or zinc.

7. The peroxide microparticle of claim 6, wherein the mean particle size (D50) of the one or more solid peroxide particles is between 0.1 µm and 8 µm, and preferably between 0.1 µm and 4 µm.

8. A topical composition comprising the encapsulated peroxide particles according to claim 5 or a plurality of the peroxide microparticles according to claim 6 or 7.

9. The topical composition of claim 8, wherein the topical composition is a cream, gel or lotion.

10. The topical composition of any one of claims 8 or 9, further comprising one or more additives selected from the group consisting of thickeners, preservatives, antioxidants, fragrances, emulsifiers, moisturizing agents, emollients, sequestering agents, surfactants, fillers, sunscreen agents, colorants, and combinations thereof.

11. The topical composition of any one of claims 8 to 10, for use in the treatment of a skin disorder.

12. The topical composition of claim 11, wherein the skin disorder is acne.

13. The topical composition of claim 11 or 12, wherein the topical composition is for being applied to skin.

## Patentansprüche

1. Verfahren zum Verkapseln eines festen Benzoylperoxids, das umfasst:
- Dispergieren von festen Benzoylperoxidpartikeln in einer wässrigen Dispersion aus einem oder mehreren einwertigen Fettsäuresalzen, um eine Dispersion aus Benzoylperoxid/einwertigen Fettsäuresalzen zu bilden, wobei die Benzoylperoxidpartikel eine mittlere Partikelgröße im Durchmesser (D50) von 10 µm oder weniger aufweisen; und
- danach Verkapseln der festen Benzoylperoxidpartikel durch Umsetzen des einen oder der mehreren einwertigen Fettsäuresalze mit einem oder mehrere mehrwertigen Salzen von Calcium, Magnesium, Aluminium, Silber und/oder Zink, wodurch verkapselte feste Peroxidpartikel von Benzoylperoxid gebildet werden.

2. Verfahren nach Anspruch 1, das ferner das Homogenisieren der Dispersion aus Peroxid/einwertigen Fettsäuresalzen umfasst, wobei das Homogenisieren bewirkt, dass die einwertigen Fettsäuresalze Mizellen um die festen Peroxidpartikel herum bilden.

3. Verfahren nach Anspruch 1 oder 2, wobei die einwertigen Fettsäuresalze aus der Gruppe ausgewählt sind, die aus Natrium- und Kaliumsalzen von Omega-3-Fettsäuren, Natrium- und Kaliumsalzen von Omega-6-Fettsäuren, Natrium- und Kaliumsalzen von Gamma-Linoleinsäure, Natrium- und Kaliumsalzen von Retinsäure, Natrium- und Kaliumsalzen von Rizinolsäure, Natrium- und Kaliumsalzen von Caprylsäure, Natrium- und Kaliumsalzen von Caprinsäure, Natrium- und Kaliumsalzen von Laurinsäure, Natrium- und Kaliumsalzen von Myristinsäure, Natrium- und Kaliumsalzen von Palmitinsäure, Natrium- und Kaliumsalzen von Stearinsäure, Natrium- und Kaliumsalzen von Arachinsäure, Natrium- und Kaliumsalzen von Behensäure, Natrium- und Kaliumsalzen von Erucasäure, Natrium- und Kaliumsalzen von Lignostearinsäure, Natrium- und Kaliumsalzen von Cerotinsäure, Natrium- und Kaliumsalzen von Ölsäure, Natrium- und Kaliumsalzen von Elaidinsäure, Natrium- und Kaliumsalzen von Linolsäure, Natrium- und Kaliumsalzen von Undecylensäure, Natrium- und Kaliumsalzen von Alpha-Linolensäure, Natrium- und Kaliumsalzen von Arachidonsäure, Natrium- und Kaliumsalzen von Ascorbinsäure, Natrium- und Kaliumsalzen von Eleostearinsäure und Kombinationen davon besteht.

4. Verfahren nach einem der Ansprüche 1 bis 3, wobei die mittlere Partikelgröße (D50) der Peroxidpartikel zwischen 0,1 µm und 10 µm und vorzugsweise zwischen 0,1 µm und 4 µm beträgt.

5. Verkapselte Peroxidpartikel, die gemäß dem Verfahren nach einem der Ansprüche 1 bis 4 hergestellt werden.

6. Benzoylperoxidmikropartikel, der umfasst:
- einen oder mehrere feste Benzoylperoxidpartikel, die innerhalb eines oder mehrerer mehrwertiger Fette verkapselt sind,
- wobei der eine oder die mehreren festen Benzoylperoxidpartikel eine mittlere Partikelgröße im Durchschnitt (D50) von 10 µm oder weniger aufweisen, und
- wobei das eine oder die mehreren mehrwertigen Fettsäuresalze aus der Gruppe ausgewählt sind, die aus Salzen von Omega-3-Fettsäuren, Salzen von Omega-6-Fettsäuren, Salzen von Gamma-Linoleinsäure, Salzen von Retinsäure, Salzen von Rizinolsäure, Salzen von Caprylsäure, Salzen von Caprinsäure, Salzen von Laurinsäure, Salzen von Myristinsäure, Salzen von Palmitinsäure, Salzen von Stearinsäure, Salzen von Arachinsäure, Salzen von Behensäure, Salzen von Erucasäure, Salzen von Lignostearinsäure, Salzen von Cerotinsäure, Salzen von Ölsäure, Salzen von Elaidinsäure, Salzen von Linolsäure, Salzen von Undecylensäure, Salzen von Alpha-Linolensäure, Salzen von Arachidonsäure, Salzen von Ascorbinsäure, Salzen von Eleostearinsäure und Kombinationen davon besteht, wobei die Salze Salze von Calcium, Magnesium, Aluminium, Silber und/oder Zink sind.

7. Peroxidmikropartikel nach Anspruch 6, wobei die mittlere Partikelgröße (D50) des einen oder der mehreren festen Peroxidpartikel zwischen 0,1 µm und 8 µm und vorzugsweise zwischen 0,1 µm und 4 µm beträgt.

8. Topische Zusammensetzung, die die verkapselten Peroxidpartikel nach Anspruch 5 oder eine Mehrzahl der Peroxidmikropartikel nach Anspruch 6 oder 7 umfasst.

9. Topische Zusammensetzung nach Anspruch 8, wobei die topische Zusammensetzung eine Creme, ein Gel oder eine Lotion ist.

10. Topische Zusammensetzung nach einem der Ansprüche 8 oder 9, die ferner einen oder mehrere Zusatzstoffe umfasst, die aus der Gruppe ausgewählt sind, die aus Verdickungsmitteln, Konservierungsmitteln, Antioxidationsmitteln, Duftstoffen, Emulgatoren, Feuchthaltemitteln, Weichmachern, Sequestriermitteln, Tensiden, Füllstoffen, Sonnenschutzmitteln, Farbstoffen und Kombinationen davon besteht.

11. Topische Zusammensetzung nach einem der Ansprüche 8 bis 10 zur Verwendung bei der Behandlung einer Hautstörung.

12. Topische Zusammensetzung nach Anspruch 11, wobei die Hautstörung Akne ist.

13. Topische Zusammensetzung nach Anspruch 11 oder 12, wobei die topische Zusammensetzung zum Auftragen auf die Haut vorgesehen ist.

## Revendications

1. Procédé d'encapsulation d'un peroxyde de benzoyle solide, comprenant :
- la dispersion de particules de peroxyde de benzoyle solide dans une dispersion aqueuse d'un ou plusieurs sels d'acides gras monovalents pour former une dispersion de peroxyde de benzoyle/sel d'acide gras monovalent, dans laquelle les particules de peroxyde de benzoyle ont une granulométrie moyenne (D50) de 10 µm ou moins ; et
- ensuite l'encapsulation des particules de peroxyde de benzoyle solide par réaction du ou des sels d'acide gras monovalents avec un ou plusieurs sels multivalents de calcium, magnésium, aluminium, argent et/ou zinc, formant ainsi des particules de peroxyde solides de peroxyde de benzoyle encapsulées.

2. Procédé selon la revendication 1, comprenant en outre l'homogénéisation de la dispersion de peroxyde/sel d'acides gras monovalents, dans lequel l'homogénéisation provoque la formation par les sels d'acide gras monovalents de micelles autour des particules de peroxyde solide.

3. Procédé selon la revendication 1 ou 2, dans lequel les sels d'acide gras monovalents sont choisis dans le groupe constitué par les sels sodiques et potassiques d'acides gras oméga-3, les sels sodiques et potassiques d'acides gras oméga-6, les sels sodiques et potassiques d'acide gamma-linolénique, les sels sodiques et potassiques d'acide rétinoïque, les sels sodiques et potassiques d'acide ricinoléique, les sels sodiques et potassiques d'acide caprylique, les sels sodiques et potassiques d'acide caprique, les sels sodiques et potassiques d'acide laurique, les sels sodiques et potassiques d'acide myristique, les sels sodiques et potassiques d'acide palmitique, les sels sodiques et potassiques d'acide stéarique, les sels sodiques et potassiques d'acide arachidique, les sels sodiques et potassiques d'acide béhénique, les sels sodiques et potassiques d'acide érucique, les sels sodiques et potassiques d'acide lignostéarique, les sels sodiques et potassiques d'acide cérotique, les sels sodiques et potassiques d'acide oléique, les sels sodiques et potassiques d'acide élaïdique, les sels sodiques et potassiques d'acide linoléique, les sels sodiques et potassiques d'acide undécylénique, les sels sodiques et potassiques d'acide alpha-linolénique, les sels sodiques et potassiques d'acide arachidonique, les sels sodiques et potassiques d'acide ascorbique, les sels sodiques et potassiques d'acide éléostéarique, et leurs combinaisons.

4. Procédé selon l'une quelconque des revendications 1 à 3, dans lequel la granulométrie moyenne (D50) des particules de peroxyde est comprise entre 0,1 µm et 10 µm, et de préférence entre 0,1 µm et 4 µm.

5. Particules de peroxyde encapsulées préparées selon le procédé de l'une quelconque des revendications 1 à 4.

6. Microparticule de peroxyde de benzoyle comprenant :
- une ou plusieurs particules de peroxyde de benzoyle solide encapsulées dans un ou plusieurs sels d'acide gras multivalents,
- dans laquelle la ou les particules de peroxyde de benzoyle solide ont une granulométrie moyenne (D50) de 10 µm ou moins, et
- dans laquelle le ou les sels d'acide gras multivalents sont choisis dans le groupe constitué par les sels d'acides gras oméga-3, les sels d'acides gras oméga-6, les sels d'acide gamma-linolénique, les sels d'acide rétinoïque, les sels d'acide ricinoléique, les sels d'acide caprylique, les sels d'acide caprique, les sels d'acide laurique, les sels d'acide myristique, les sels d'acide palmitique, les sels d'acide stéarique, les sels d'acide arachidique, les sels d'acide béhénique, les sels d'acide érucique, les sels d'acide lignostéarique, les sels d'acide cérotique, les sels d'acide oléique, les sels d'acide élaïdique, les sels d'acide linoléique, les sels d'acide undécylénique, les sels d'acide alpha-linolénique, les sels d'acide arachidonique, les sels d'acide ascorbique, les sels d'acide éléostéarique, et leurs combinaisons, lesdits sels étant des sels de calcium, magnésium, aluminium, argent et/ou zinc.

7. Microparticule de peroxyde selon la revendication 6, dans laquelle la granulométrie moyenne (D50) de la ou des particules de peroxyde solide est comprise entre 0,1 µm et 8 µm, et de préférence entre 0,1 µm et 4 µm.

8. Composition à usage topique comprenant les particules de peroxyde encapsulées selon la revendication 5 ou une pluralité de microparticules de peroxyde selon la revendication 6 ou 7.

9. Composition à usage topique selon la revendication 8, laquelle composition à usage topique est une crème, un gel ou une lotion.

10. Composition à usage topique selon l'une quelconque des revendications 8 et 9, comprenant en outre un ou plusieurs additifs choisis dans le groupe constitué par les épaississants, les conservateurs, les antioxydants, les parfums, les émulsifiants, les agents hydratants, les émollients, les agents séquestrants, les tensioactifs, les charges, les agents de protection solaire, les colorants, et leurs combinaisons.

11. Composition à usage topique selon l'une quelconque des revendications 8 à 10, pour une utilisation dans le traitement d'un trouble cutané.

12. Composition à usage topique selon la revendication 11, dans laquelle le trouble cutané est l'acné.

13. Composition à usage topique selon la revendication 11 ou 12, dans laquelle la composition à usage topique est pour être appliquée sur la peau.
